(19) Europäisches Patentamt
European Patent Office
Office européen des brevets

(11) Numéro de publication : 0 283 379 B1

(12) # FASCICULE DE BREVET EUROPEEN

(45) Date de publication du fascicule du brevet : 17.04.91 Bulletin 91/16

(51) Int. Cl.$^5$ : C07C 2/84, B01J 23/02, B01J 23/10

(21) Numéro de dépôt : 88400534.9

(22) Date de dépôt : 07.03.88

(54) **Procédé de conversion du méthane en hydrocarbures supérieurs.**

(30) Priorité : 16.03.87 FR 8703713

(43) Date de publication de la demande : 21.09.88 Bulletin 88/38

(45) Mention de la délivrance du brevet : 17.04.91 Bulletin 91/16

(84) Etats contractants désignés : DE GB IT NL

(56) Documents cités :
EP-A- 0 196 541
US-A- 4 499 323
US-A- 4 499 324

(73) Titulaire : INSTITUT FRANCAIS DU PETROLE
4, Avenue de Bois-Préau
F-92502 Rueil-Malmaison (FR)

(72) Inventeur : Mimoun, Hubert
34, rue Hippolyte Bisson
F-92500 Rueil Malmaison (FR)
Inventeur : Bonnaudet, Serge
4, rue de Cheroy
F-75017 Paris (FR)
Inventeur : Cameron, Charles
28, rue Henri Barbusse
F-75005 Paris (FR)
Inventeur : Robine, Alain
10, rue Massena
F-92500 Rueil Malmaison (FR)

EP 0 283 379 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de conversion oxydante du méthane ou du gaz naturel en hydrocarbures supérieurs, caractérisé en ce que l'on fait passer un mélange de méthane et d'oxygène au contact d'un catalyseur solide constitué d'au moins un alkoxyde de métal alcalin déposé sur un support, tel que, par exemple, un oxyde de métal alcalino terreux ou de métal de terre rare ou un mélange de ces oxydes, solide à la température de réaction.

Le gaz naturel est une matière première fossile abondante constituée essentiellement de méthane, dont les réserves prouvées actuellement, de l'ordre de $10^{14}$ $m^3$ représentent environ 50 années de consommation mondiale. Les gisements de gaz se trouvent souvent très éloignés des lieux de consommation, ce qui nécessite un transport coûteux par réseaux de canalisations ou par navires méthaniers dans lesquels le gaz naturel est transporté sous forme de gaz liquéfié à −162°C.

La transformation du méthane en homologues hydrocarbonés supérieurs liquides sur le site de production serait donc infiniment avantageuse. Elle se heurte toutefois au problème de l'inertie chimique considérable du méthane et à la thermodynamique peu favorable de sa transformation.

La plupart des méthodes de transformation du gaz naturel passent par sa conversion en gaz de synthèse ($CO + H_2$), laquelle représente une réaction très endothermique grosse consommatrice d'énergie. Le gaz de synthèse est généralement converti en méthanol, lequel est ensuite déshydraté en éther diméthylique qui est lui-même converti en hydrocarbures aromatiques sur Zeolite ZSM5.

La transformation directe du méthane en éthylène serait un objectif hautement désirable, l'éthylène pouvant servir de matière première pour de nombreuses synthèses de produits importants.

La réaction de couplage oxydant du méthane peut s'écrire :

$$CH_4 \xrightarrow{O_2} CH_3\text{-}CH_3 + CH_2\text{=}CH_2 + CO_2 + H_2O$$

et peut s'effectuer, selon la nature du catalyseur utilisé, en mode simultané (balayage du catalyseur par un mélange $CH_4 + O_2$) ou séquentiel (oxydation du méthane en hydrocarbures supérieurs par le catalyseur, suivie de la réoxydation du catalyseur par l'oxygène de l'air).

– Keller et Bhasin (J. Catalysis 1982, 73, 9) ont montré que les oxydes de plomb, d'étain, de bismuth, de tellure et de cadmium déposés sur alumine catalysent le couplage du méthane en mélange éthylène-éthane à 750°C et pression atmosphérique en mode séquentiel. La conversion du méthane est très faible (2%) et la sélectivité en $C_{2+}$ est de l'ordre de 50-55%.

– Hinsen et Baerns (Chem. Zeitung, 1983, 107, 223) ont réalisé le couplage oxydant du méthane en mode simultané ou séquentiel en utilisant des catalyseurs à base d'oxyde de plomb sur silice ou alumine. La sélectivité en $C_{2+}$ est de 50-55%. Le catalyseur se désactive rapidement à cause de la perte de PbO du catalyseur.

– Otsuka et collaborateurs (Chem. Letters, 1985, 449) ont utilisé une série d'oxydes de terres rares parmi lesquels l'oxyde de samarium est le plus sélectif.

– on peut également citer les brevets US 4 499 323, 4 499 324, 4 489 215, 4 495 374, 4 443 648, 4 443 645, 4 444 984, 4 443 649, 4 443 647, 4 443 646, 4 523 050, 4 523 049 concernant l'utilisation d'oxydes métalliques aussi divers que $Pr_6O_{11}$, $CeO_2$, $RuO_2$, $BaO\text{-}MnO_2/SiO_2$, $GeO_2$, $In_2O_3$, $Mn_3O_4$, PbO, $Bi_2O_3$, $MnSiO_3$ pour le couplage oxydant du méthane à 700-800°C.

– Lunsford et collaborateurs (J. Am. Chem. Soc., 1985, 107, 58) ont montré que l'oxyde (ou le carbonate) de lithium déposé sur magnésie catalyse de manière efficace le couplage oxydant du méthane en mélange éthylène-éthane à 700-750°C et pression atmosphérique.

Un des inconvénients de l'utilisation des catalyseurs sus-désignés tient à la température élevée, généralement supérieure à 700°C, à laquelle ils opèrent. Ces conditions de température élevée provoquent des phénomènes indésirables tels que frittage des catalyseurs, perte en constituants actifs volatils et dépôt de coke.

Un autre inconvénient des catalyseurs précités tient à leur sélectivité insuffisante en produits de couplage tels que l'éthane et l'éthylène, parmi lesquels l'éthane est le plus souvent le constituant prépondérant, alors que l'éthylène constitue le produit le plus recherché.

On a maintenant découvert une nouvelle procédure de préparation de catalyseurs permettant d'obtenir une activité et une sélectivité accrues en produits de couplage. Grâce à cette plus grande activité, ils peuvent être utilisés à des températures habituellement moins élevées que celle utilisées dans l'art antérieur.

Cette procédure consiste à imprégner en solution alcoolique un support constitué d'au moins un oxyde réfractaire (c'est à dire demeurant à l'état solide dans les conditions de préparation et d'utilisation) par au moins un alkoxyde de métal alcalin ou alcalino-terreux, l'oxyde réfractaire étant de préférence choisi parmi les oxydes des métaux alcalino-terreux et des métaux des terres rares.

On peut citer à titre d'exemples de supports utilisables dans la présente invention, à titre individuel ou en mélanges ou combinaisons, l'oxyde de magnésium, l'oxyde de calcium, l'oxyde de strontium, l'oxyde de baryum, l'oxyde de scandium, l'oxyde de lanthane, l'oxyde de samarium, l'oxyde de praséodyne, ou tout autre oxyde de terre rare. L'alkoxyde de métal alcalin déposé sur le support à pour formule MOR, dans laquelle M est un métal alcalin tel que le lithium, le sodium, le potassium, le rubidium, le césium et R un groupement hydrocarbyle, par exemple alkyle, aryle, aralcoyle ou alkylaryle comprenant usuellement de 1 à 20 atomes de carbone. On peut citer à titre d'exemples non limitatifs les méthylates, éthylates, propylates, butylates, phenoxydes de lithium, sodium, potassium, rubidium et césium. On préfère les alkoxydes de lithium et particulièrement le méthylate de lithium.

L'alkoxyde de métal alcalino-terreux a pour formule $M'(OR)_2$ dans laquelle M' est un métal alcalino-terreux tel que le beryllium, le magnésium, le calcium, le strontium et le baryum, et R un groupement hydrocarbyle, par exemple alkyle, aryle, aralcoyle ou alcoylaryle comprenant usuellement de 1 à 20 atomes de carbone. On peut citer à titre d'exemples non limitatifs les méthylates, éthylates, propylates, butylates de beryllium, magnésium, calcium, baryum et strontium.

Une méthode préférée de préparation des catalyseurs consiste à immerger le support dans une solution alcoolique de métal alcalin ou alcalino-terreux. Par exemple, la dissolution de lithium, de sodium ou de potassium métallique dans un alcool conduit à la formation d'une solution de l'alkoxyde de métal alcalin, qui est utilisée pour imprégner le support. On peut utiliser comme solvant alcoolique du métal alcalin, à titre non limitatif le méthanol. l'éthanol le n-propanol, les n ou isobutanols, les pentanols, hexanols, octanols, etc.

On élimine ensuite le solvant. Pour cela, la solution alcoolique d'imprégnation contenant le support peut, par exemple être évaporée lentement dans un évaporateur rotatif pour éliminer l'alcool en excès, puis séchée sous vide, et calcinée (décomposée) à une température d'environ 300 à 800°C de préférence 400 à 700°C, avantageusement 420-650°C.

La teneur pondérale en métal alcalin ou alcalino-terreux, par rapport au support peut très avantageusement être comprise entre 0,1 et 20%, de préférence entre 1 et 10%.

La réaction selon l'invention procède par passage d'un mélange méthane (ou gaz naturel) – oxygène au contact du catalyseur à l'état solide. Pour des raisons de sécurité, on préfère opérer avec des proportions d'oxygène, par rapport au méthane, d'au plus 10% en mole, par exemple 1-10% en mole. Toutefois, on peut dépasser cette teneur de 10%, et donc augmenter la conversion de la réaction, en opérant en présence d'un gaz inerte, tel que l'azote et donc utiliser de l'air. On peut également utiliser un diluant tel que la vapeur d'eau ou le gaz carbonique.

On peut également augmenter la conversion en utilisant plusieurs zones de réaction en série avec introduction à chaque étape d'une quantité contrôlée d'oxygène, tout en restant en dehors des limites d'explosivité.

La température de réaction est généralement comprise entre 300°C et 950°C, par exemple entre 300 et 800°C, de préférence entre 600 et 900°C, plus particulièrement entre 750 et 850°C.

La pression totale dans le réacteur peut être par exemple de 1 à 100 bars, et notamment de 1 à 10 bars. Le temps de contact est, de préférence, de $10^{-3}$ sec. à 10 sec.

## EXEMPLE 1

### 1. Préparation du catalyseur

On prépare une solution méthanolique de méthylate de lithium par dissolution de 6,4 g. de lithium métallique dans 600 ml de méthanol anhydre. On introduit alors 80 g d'oxyde de lanthane pur en poudre dans la solution. On porte le mélange à reflux du méthanol à 60°C pendant une heure, puis on évapore la solution à 80°C jusqu'à obtenir une poudre sèche de catalyseur.

La poudre est alors séchée sous vide à 200°C pendant quatre heures, puis calcinée sous azote à 550°C pendant quatre heures.

### 2. Test catalytique

On mélange intimement 2 g. de poudre de catalyseur avec 2 g. de poudre de quartz, et l'on introduit le tout dans un réacteur tubulaire en alumine frittée que l'on porte à une température de 530°C. On fait passer alors sur le catalyseur un mélange gazeux de méthane et d'oxygène avec des débits respectifs de 50 ml/minute

et 5 ml/minute.

L'analyse chromatographique du mélange à la sortie révèle que 4,5% du méthane a été converti. On a formé de l'éthylène avec une sélectivité molaire (s.m.) de 65%, de l'éthane (s.m. = 14%) et du CO + $CO_2$ (s.m. = 12%).

La conversion du méthane et les sélectivités en produits $C_2$ sont restées relativement constantes pendant 100 heures de réaction.

EXEMPLE 2 (comparatif)

On imprègne 50 grammes d'oxyde de lanthane par 500 ml d'une solution aqueuse contenant 14 g. de carbonate de lithium (même proportion de Li que dans l'exemple 1). Après évaporation de la solution sous vide à 100°C, on obtient une poudre de catalyseur que l'on sèche à 200°C pendant quatre heures, puis que l'on calcine à 550°C sous azote pendant quatre heures.

On opère comme dans l'exemple 1, en envoyant un mélange de méthane (50 ml/minute) et d'oxygène (5 ml/minute) sur un mélange de deux grammes de poudre de catalyseur avec deux grammes de poudre de quartz porté dans le réacteur tubulaire à une température de 530°C.

L'analyse chromatographique de l'effluent gazeux à la sortie indique que 3% du méthane a été converti, et qu'il s'est formé de l'éthylène (s.m. = 34%), de l'éthane (s.m. = 7%) et du $CO_2$ + CO (s.m. = 59%). La sélectivité décroît sensiblement au cours du temps.

Cet essai montre que l'imprégnation de l'oxyde de lanthane par du méthylate de lithium en solution méthanolique (exemple 1) conduit à un catalyseur plus actif et plus sélectif, que celui préparé par la voie connue en phase aqueuse ($Li_2CO_3/H_2O$).

EXEMPLE 3

On prépare un catalyseur comme dans l'exemple 1 en imprégnant 100 g d'oxyde de calcium pur CaO par une solution méthanolique de méthylate de lithium (6,4 g. de lithium métallique dissous dans 600 ml de méthanol). Après évaporation de la solution à 80°C, la poudre de catalyseur est séchée à 200°C sous vide pendant deux heures, puis calcinée sous azote à 550°C.

Deux grammes de catalyseur sont introduits dans le réacteur tubulaire qui est porté à la température de 510°C. On fait passer alors un mélange de méthane (50 ml/min) et d'oxygène (5 ml/min).

L'analyse de l'effluent gazeux montre que l'on a converti 4,5% du méthane et qu'il s'est formé de l'éthylène (s.m. = 48%), de l'éthane (s.m. = 21%), et du CO + $CO_2$ (s.m. = 30%).

EXEMPLE 4

On prépare un catalyseur en imprégnant 50 g d'oxyde de lanthane $La_2O_3$ par une solution ethanolique d'éthylate de baryum (10 g de baryum métallique dissous à reflux dans 100 ml d'éthanol sec). On porte le mélange à reflux de l'éthanol à 80°C pendant une heure, puis on évapore la solution à 100°C pour obtenir une poudre sèche de catalyseur.

La poudre est alors séchée à 200°C pendant 2 heures, puis calcinée à 600°C pendant 2 heures.

On mélange intimement 0,5 g de poudre de catalyseur avec 3 ml d'alumine tabulaire dans le réacteur selon l'exemple 1, que l'on porte à la température de 700°C.

On envoie alors un mélange de méthane (1000 ml/min) et d'oxygène (100 ml/min) au travers du lit catalytique. L'analyse chromatographique de l'effluent gazeux à la sortie indique que 17% de méthane et 100% de l'oxygène ont été convertis, et qu'il s'est formé de l'éthylène (s. m. = 36%), de l'éthane (s.m. = 42%), du $CO_2$ + CO (18%). La sélectivité et la conversion restent constantes au cours du temps.

EXEMPLE 5

On prépare un catalyseur comme dans l'exemple 4 en imprégnant 50 g d'oxyde de lanthane $La_2O_3$ par une solution éthanolique d'éthylate de strontium (1,4 g strontium métallique dissous à reflux dans 100 ml d'éthanol sec). Après imprégnation à reflux de l'éthanol pendant une heure, évaporation de l'éthanol, séchage à 200°C pendant 2 heures, et calcination à 600°C pendant 2 heures, on obtient une poudre blanche.

On mélange 0,5 g de cette poudre avec 3 ml d'alumine tabulaire, que l'on introduit dans le réacteur.

On envoie alors à travers le catalyseur un mélange de 1000 ml/min. de méthane et 150 ml/min. d'oxygène pur. L'analyse de l'effluent à la sortie indique que l'on a converti 20% du méthane et 100% de l'oxygène et qu'il s'est formé de l'éthylène (s.m. = 36%), de l'éthane (s.m. = 36%), et du CO + $CO_2$ (s.m. = 25%).

## Revendications

1. Procédé de conversion du méthane en hydrocarbures supérieurs, dans lequel on fait passer un gaz renfermant du méthane et de l'oxygène au contact d'un catalyseur, caractérisé en ce que le catalyseur est le matériau résultant de l'imprégnation d'un oxyde réfractaire par une solution d'au moins un alkoxyde de métal alcalin ou de métal alcalino-terreux, séchage et calcination, le dit oxyde réfractaire étant choisi parmi les oxydes de métaux alcalino-terreux ou de métaux des terres rares.

2. Procédé selon la revendication 1, dans lequel l'alkoxyde est un méthylate, éthylate, propylate, butylate ou phénoxyde de lithium, sodium, potassium, rubidium ou césium.

3. Procédé selon la revendication 2, dans lequel l'alkoxyde de métal est le méthylate de lithium.

4. Procédé selon l'une des revendications 1 à 3, dans lequel l'oxyde réfractaire est l'oxyde de lanthane.

5. Procédé selon l'une des revendications 1 à 3, dans lequel l'oxyde réfractaire est l'oxyde de calcium.

6. Procédé selon l'une des revendications 1 à 5, dans lequel la température de calcination est de 300 à 800°C.

7. Procédé selon l'une des revendications 1 à 6, dans lequel la teneur en métal alcalin, par rapport à l'oxyde réfractaire, est de 0,1 à 20% en poids.

8. Procédé selon l'une des revendications 1 à 7, dans lequel on opère à 300-950°C.

9. Procédé selon l'une des revendications 1 à 7, dans lequel on opère à 300-800°C.

10. Procédé selon l'une des revendications 1 à 9, dans lequel le gaz renferme l'oxygène en proportion de 1 à 10% en mole, par rapport au méthane.

11. Procédé selon la revendication 1, dans lequel l'alkoxyde est un alkoxyde de métal alcalino-terreux.

## Ansprüche

1. Verfahren zur Umsetzung von Methan zu höheren Kohlenwasserstoffen, bei dem man ein Methan und Sauerstoff enthaltendes Gas in Kontakt mit einem Katalysator zu passieren veranlaßt, dadurch gekennzeichnet, daß der Katalysator ein Material ist, das aus der Imprägnierung eines feuerfesten Oxyds durch eine Lösung wenigstens eines Alkalimetall- oder Erdalkalimetallalkoxyds, der Trocknung und Kalzinierung stammt, wobei das feuerfeste Oxyd gewählt ist aus den Oxyden der Erdalkalimetalle oder der Seltenerdmetalle.

2. Verfahren nach Anspruch 1, bei dem das Alkoxyd ein Methylat, Ethylat, Propylat, Butylat oder Phenoxyd von Lithium, Natrium, Kalium, Rubidium oder Cäsium ist.

3. Verfahren nach Anspruch 2, bei dem das Metallalkoxyd Lithiummethylat ist.

4. Verfahren nach einem der Ansprüche 1 bis 3, bei dem das feuerfeste Oxyd Lanthanoxyd ist.

5. Verfahren nach einem der Ansprüche 1 bis 3, bei dem das feuerfeste Oxyd Kalziumoxyd ist.

6. Verfahren nach einem der Ansprüche 1 bis 5, bei dem die Kalzinierungstemperatur 300 bis 800°C beträgt.

7. Verfahren nach einem der Ansprüche 1 bis 6, bei dem der Gehalt an Alkalimetall bezogen auf das feuerfeste Oxyd 0.1 bis 20 Gew.-% beträgt.

8. Verfahren nach einem der Ansprüche 1 bis 7, bei dem man bei 300-950°C arbeitet.

9. Verfahren nach einem der Ansprüche 1 bis 7, bei dem man bei 300-800°C arbeitet.

10. Verfahren nach einem der Ansprüche 1 bis 9, bei dem das Gas Sauerstoff in einem Anteil von 1 bis 10 Mol-% bezogen auf das Methan enthält.

11. Verfahren nach Anspruch 1, bei dem das Alkoxyd ein Erdalkalimetallalkoxyd ist.

## Claims

1. Process for the conversion of methane into higher hydrocarbons, in which a gas containing methane and oxygen is brought into contact with a catalyst, characterized in that the catalyst is the material resulting from the impregnation of a refractory oxide by a solution of at least one alkaline earth metal or alkali metal alkoxide, drying and calcining, said refractory oxide being chosen from among rare earth metal or alkaline earth metal oxides.

2. Process according to claim 1, wherein the alkoxide is a lithium, sodium, potassium, rubidium or cesium methylate, ethylate, propylate, butylate or phenoxide.

3. Process according to claim 2, wherein the metal alkoxide is lithium methylate.

4. Process according to one of the claims 1 to 3, wherein the refractory oxide is lanthanum oxide.

5. Process according to one of the claims 1 to 3, wherein the refractory oxide is calcium oxide.

6. Process according to one of the claims 1 to 5, wherein the calcination temperature is 300 to 800°C.

7. Process according to one of the claims 1 to 6, wherein the alkali metal content, based on the refractory oxide, is 0.1 to 20% by weight.

8. Process according to one of the claims 1 to 7, wherein working takes place at 300 to 950°C.

9. Process according to one of the claims 1 to 7, wherein working takes place at 300 to 800°C.

10. Process according to one of the claims 1 to 9, wherein the gas contains oxygen in a proportion of 1 to 10 molar %, based on the methane.

11. Process according to claim 1, wherein the alkoxide is an alkaline earth metal alkoxide.